# EUROPEAN PATENT APPLICATION

(11) **EP 4 548 865 A1**
(43) Date of publication of application: **07.05.2025**
(21) Application number: 23208057.2
(22) Date of filing: 06.11.2023
(51) Int. Cl.: A61B 17/24, A61B 17/34, A61N 1/05, A61N 1/375

(54) **SYSTEM AND METHOD FOR IMPLANTING A STIMULATION LEAD INTO A SUBJECT'S BODY**

(71) Applicant: Man & Science SA, 1435 Mont-St-Guibert (BE)
(72) Inventor: FOSSUM BRATBAK, Daniel, 7023 Trondheim (NO); MEVEL, Hervé, 1450 Chastre (BE); VAN BUYTEN, Jean-Pierre, 9250 Waasmunster (BE)
(74) Representative: Patentanwälte Olbricht Buchhold Keulertz

(57) **Abstract**

The invention disclosed herein relates to a system (1) for implanting a neurostimulation lead (2) in a subject's head and facial tissue (3), the system (1) comprising: a stimulation lead (2) configured for implantation in said tissue (3); an introducer (4) configured for temporary insertion into said tissue (3) and comprising a Tube portion (5); a tunneling tool (6) configured for temporary insertion into said tissue (3) and comprising a shaft (7), a shaft tip (9) releasably mounted to the shaft (7), and a pull suture (8) connected to the releasable shaft tip (9); a first trocar (10) configured for insertion into the introducer (4), the first trocar (10) comprising a receiving structure (11) for receiving and retaining the shaft tip (9) of the tunneling tool (6); a guide tube (12) configured for insertion into the introducer (4); a second trocar (13) configured for insertion into the guide tube (12); and a lead holder (14) configured for inserting the stimulation lead (2) through the guide tube (12) into said tissue. The invention further relates to a method of implanting a stimulation lead (2) in a subject's head and facial tissue (3) using said system (1).

## Description

### Technical field

The invention disclosed herein relates to a system for implanting a neurostimulation lead into a subject's head and facial tissue, e.g. the sphenopalatine ganglion, for the treatment of head and/or facial pain. Furthermore, the invention relates to a method of implanting a neurostimulation lead into the subject's head and facial tissue using said system.

### Background

Neural modulation, i.e. electrical stimulation of nerves, presents the opportunity to treat many physiological conditions and disorders by interacting with the body's own natural neural processes. Neural stimulation includes inhibition (e.g. blockage), modulation, modification, regulation, or therapeutic alteration of activity, electrical or chemical, in the central, peripheral, or autonomic nervous system.

By modulating the activity of the nervous system, for example through the stimulation of nerves or the blockage of nerve signals, several different goals may be achieved. Motor neurons may be stimulated at appropriate times to cause muscle contractions. Sensory neurons may be blocked, for instance to relieve pain, or stimulated, for instance to provide a signal to a subject. In other examples, modulation of the autonomic nervous system may be used to adjust various involuntary physiological parameters, such as heart rate and blood pressure. Neural modulation may provide the opportunity to treat several diseases or physiological conditions, a few examples of which are described in detail below.

Typically, neural stimulators deliver therapy in the form of electrical pulses and include two or more electrodes in a proximity of the target location, such as a specific nerve or section thereof. Electrical stimulation is adjustable through various parameters, such as the polarity of electrode(s), voltage or current amplitudes, pulse frequency, pulse width, configuration and selection of electrodes and others, as these define the electrical stimulation therapy to be delivered to the user in need of therapy. Such parameters may be preprogrammed or programmable to deliver the desired stimulation and result desired from the stimulation therapy.

Head or facial pain (e.g. migraine headaches or trigeminal neuropathy) is among the conditions to which neural stimulation may be applied. Conventional treatments typically involve the use of analgesics of varying strengths. However, due to neural involvement in the sensation of pain, methods and devices aimed at neural stimulation may offer a different solution. Neural stimulation may also be an effective solution to other conditions, for example, cluster headaches. The foregoing are just a few examples of conditions to which neuromodulation may be of benefit, however embodiments of the invention described hereafter are not necessarily limited to treating only the above-described conditions.

Some methods used in the treatment of head pain conditions comprise manipulation and/or stimulation of the sphenopalatine ganglion (SPG). The SPG is an extracranial neuronal center found in pterygopalatine fossa (i.e. behind the nose). It consists of parasympathetic neurons that, among others, innervate the middle cerebral and anterior cerebral blood vessels, and the facial blood vessels. Manipulation of the SPG is mostly performed in treatments of severe headaches, such as cluster headaches or chronic migraines. The SPG has the shape of a pyramid with a mean diameter of 3.5 mm. It is suspended from the maxillary nerve by the sphenopalatine nerves. Preganglionic parasympathetic fibers form the nervus intermedius of the facial nerve synapse with postganglionic fibers innervating the lacrimal gland, mucosa of the sinonasal cavity and cerebral blood vessels. Postganglionic sympathetic fibers from the superior cervical ganglion pass through the ganglion as well as sensory nerves from the maxillary nerve that innervates the palate and the epipharynx. The SPG is located in the sphenopalatine fossa (SF) and narrows down inferiorly with the apex pointing downwards into the greater palatine canal. The SF is bound superiorly by the infraorbital fissure, laterally by the pterygomaxillary fissure, medially by the palatine bone, posteriorly by the pterygoid plates, anteriorly by the posterior wall of the maxillary sinus and inferiorly by the palatine canal. Additionally, it communicates with the nasal cavity through the sphenopalatine foramen and the middle cranial fossa through the Vidian canal and foramen rotundum. It can be divided in three compartments, an anterior compartment containing mainly blood vessels, a middle compartment containing mainly adipose tissue, and a posterior compartment containing mainly neural structures.

Correct implantation of the stimulation lead is a challenge because the SPG is a deep structure and is therefore difficult to see. To overcome this problem, implantation and fixation of the stimulator is usually performed intraorally via open surgery. Once implanted, however, it is important that the lead is also positioned correctly, i.e. in its desired orientation. This may be achieved by live fluoroscopy.

A system and a method for implantation of a SPG stimulator are for example known from US2012290057A1. The known methods of implantation of stimulators for SPG stimulation and the systems used have several disadvantages. For example, the known systems require open surgery and more invasive steps, leading to visible scarring in the patient's face or on the patient's head. In addition, it may be very difficult to correctly position a stimulation lead after implantation using the known systems. Incorrect positioning of the lead may result in poor treatment effect of the subsequent stimulation therapy or increased risk of negative side effects. However, if live fluoroscopy is used during implantation, both the surgeon and patient are exposed to high amounts of radiation.

The objective technical problem of the present invention is to eliminate the disadvantages of the prior art and to provide a system for implantation of a stimulation lead in a head and facial tissue of a subject that eliminates the need for open surgery. Likewise, a minimally-invasive method of implantation of the stimulation lead using said system should be provided.

### General description of the invention

Main features of the system as described herein are specified by claim 1. Special embodiments or beneficial variants of the system are presented in claims 2 to 17. Main features of the method as described herein are specified by claim 18 Special embodiments or beneficial variants of the methods are presented in claims 19 to 23.

Disclosed herein is a system for implanting a neurostimulation lead in a subject's head and facial tissue, e.g. near the SPG, and connecting it with an implanted pulse generator (IPG), wherein the system comprises: a stimulation lead configured for implantation in said tissue; an introducer configured for temporary insertion into said tissue and comprising a tube portion; a tunneling tool configured for temporary insertion into said tissue and comprising a shaft, a shaft tip releasably mounted to the shaft, and a pull suture connected to the releasable shaft tip; a first trocar configured for insertion into the introducer, the first trocar comprising a receiving structure for receiving and retaining the releasable shaft tip of the tunneling tool; a guide tube configured for insertion into the introducer; a second trocar configured for insertion into the guide tube; and a lead holder configured for inserting the stimulation lead through the guide tube into said tissue.

The system allows for easy and safe implantation and in particular precise placement of a neurostimulation lead in a subject's head and facial tissue, since the procedure can be readily performed by a wide range of different physicians, including pain physicians and neurologists. With the system as described herein, the stimulation lead can be implanted with high accuracy, allowing for high efficacy of the stimulation therapy. Furthermore, an accurately placed lead is unobtrusive to the patient and more resilient against dislocation. Moreover, removing the lead only requires moderate effort. The lead tunneling procedure to behind the ear allows for connection of the proximal end of the lead with an already implanted IPG, e.g. with an IPG implanted in the vicinity of the occipital nerve. Thus, the system that can be combined with additional leads to potentially target more than one nerve structure associated with headache pathophysiology.

Together with the introducer, the first trocar is configured for making a puncture in a facial region of the subject to a certain tissue depth, the facial region preferably being the subject's cheek. Once removed from the introducer, i.e. from the facial tissue, the guide tube with the second trocar may be inserted into the introducer and thereby into the puncture created by the first trocar. The second trocar is configured in such a way, that it reaches a bigger tissue depth than the first trocar. In particular, the second trocar can reach the tissue surrounding the SPG or the sphenopalatine fossa. Thus, once the second trocar is removed from the introducer, the subject has an opening or channel reaching from a puncture in his skin to the tissue surrounding the SPG. This is particularly advantageous, since the SPG is a relatively deep tissue that is not easily identified visually.

The tunneling tool may further be bendable in such a way that it takes on and holds a desired shape or form. This way, the tunneling tool may be bent prior to the tunneling procedure in order to better match the subject's tissue or anatomy. For example, the shaft of the tunneling tool may be calibrated using a navigation guidance system before insertion to have up-to-date geometry.

According to a further embodiment, the releasable shaft tip of the tunneling tool may be configured for snapping into the receiving structure of the first trocar. This way, since the shaft tip of the tunneling tool is connected to the pull suture, the pull suture will be connected to the first trocar once the shaft tip has snapped into the receiving structure of the first trocar. Preferably, the receiving structure may comprise several snap-in openings arranged along a longitudinal axis of the first trocar. Such a pattern of longitudinally arranged snap-in openings has the advantage that the shaft tip of the tunneling tool and the receiving structure of the first trocar may meet and snap into each other at different depths after insertion of the tunneling tool and the first trocar, respectively. In other words, the pattern of longitudinally arranged snap-in openings compensates for different insertion depths in the subject's tissue.

According to a further embodiment, the tube portion of the introducer may be configured for slidably receiving the guide tube. In particular, the tube portion of the introducer may be configured for slidably receiving the guide tube in a flush fitting manner. The tube portion of the introducer and/or the guide tube may furthermore be shaped in such way that the guide tube may only fit inside the tube portion of the introducer in one orientation, thus preventing the guide tube from spinning around its longitudinal axis when inserted in the introducer. Likewise, the introducer may be configured for slidably receiving the first trocar. In particular, the introducer may be configured for slidably receiving the first trocar in a flush fitting manner. The introducer and/or the first trocar may furthermore be shaped in such way that the first trocar may only fit inside the introducer in one orientation, thus preventing the first trocar from spinning around its longitudinal axis. This way, a steady insertion of the guide tube and the first trocar can be guaranteed. Furthermore, alignment of certain structures, such as slot-like openings, of the introducer, the guide tube and the first trocar, may be achieved when said tools are only insertable into each other in a certain orientation.

According to a further embodiment, the guide tube may also be configured for slidably receiving the lead holder. The guide tube and/or the shaft portion of the lead holder may furthermore be shaped in such way that the lead holder may only be inserted into the guide tube in one particular orientation, thus preventing the lead holder from spinning around its longitudinal axis when inserted in the guide tube.

According to a further embodiment, the tube portion of the introducer may comprise a first slot-like opening that proceeds along a longitudinal axis of the tube portion. This allows, among other things, the shaft tip of the tunnelling tool to meet and connect with the receiving structure of the first trocar when the first trocar is inserted in the introducer.

According to a further embodiment, the guide tube may comprise a second slot-like opening that proceeds along a longitudinal axis of the guide tube. The slot-like opening of the guide tube may be particularly advantageous once the lead holder is inserted into the guide tube. In particular, the slot-like opening of the guide tube facilitates removal of the guide tube from the introducer while at the same time leaving the lead holder inserted in the tube portion of the introducer.

According to a further embodiment, the introducer may comprise a lock mechanism for locking the guide tube and/or the lead holder at a desired position or depth in the introducer when the guide tube and/or the lead holder reached its desired position in the subject's head and facial tissue. The lock mechanism of the guide tube for locking the insertion depth guide tube and/or the lead holder may for example comprise a ratchet-like mechanism. Locking the guide tube and/or the lead holder significantly facilitates implantation, since the physician is no longer required to hold guide tube or the lead holder in position with one of their hands, thus allowing them to perform other steps with two free hands.

According to a further embodiment, the introducer may comprise a disk having an adhesive portion wherein the adhesive portion of the disk may be configured for attachment to the subject's skin. Attaching the introducer to the subject's skin using the adhesive portion of the disk is advantageous, since it locks the position, i.e. the depth and the rotational angle of the introducer with respect to the subject's face, thereby allowing the physician two perform other task with both hands free. Furthermore, it is beneficial when the introducer is locked in position for all steps, in particular since the implantation procedure may take a couple of minutes and a shift, slip or rotation of the introducer may lead to inaccurate positioning of the implantable lead. Preferably, the disk may be tiltable with respect to a longitudinal axis of the tube portion and/or rotatable around the longitudinal axis of the tube portion of the introducer, wherein the disk may be in particular tiltable and/or rotatable in discrete increments. This way, the introducer may be locked in many different positions with respect to the subject's cheek. For example, the introducer may be inserted into the subject's cheek in an angle. With a tiltable disk, the introducer can still be attached to the patient's skin.

According to a further embodiment, the stimulation lead may comprise a proximal end and a distal end, wherein the proximal end is configured for implantation in the proximal target tissue and wherein the distal end is configured for implantation in the distal target tissue.

According to a further embodiment, the stimulation lead may, at its proximal end, comprise a connecting structure to which the pull suture is connectable, wherein the connecting structure is preferably a looped strand. Preferably, the pull suture may simply be tied to the connecting structure of in a knot. The stimulation lead may comprise a strain relief running internally through the lead and connected to the connecting structure. The strain relief protects the integrity of the stimulation lead, i.e. it protects the lead's internal wires from damaging while the proximal end of the lead is pulled in the direction of the tunneling incision behind the ear.

According to a further embodiment, the stimulation lead may comprise at least one pair of tines or anchors located towards its distal end. The tines can engage with the distal target tissue and guarantee that the stimulation lead stays in position after implantation. The pairs of tines can be located radially around the distal end of the lead, wherein the individual tines are preferably spaced around the distal end equidistant from each other, thus distributing the anchoring forces equally around the distal end of the lead.

Between its two ends, the stimulation lead comprises a middle portion which may comprise a scale having markings (e.g. Millimeters and/or Centimeters). The scale facilitates correct implantation of the lead to its desired position (i.e. correct insertion depth of the distal end of the lead).

According to a further embodiment, the stimulation lead may comprise at least one connector electrode at its proximal end and at least one stimulation electrode at its distal end, wherein the stimulation lead preferably comprises at least three connector electrodes at its proximal end and at least three stimulation electrodes at its distal end. The stimulation electrodes are configured for neurostimulation and can generate an electromagnetic field at the distal target tissue. According to a further embodiment, the at least one connector electrode and/or the at least one stimulation electrode may be a ring electrode. The connector electrodes are configured for connection with an already implanted IPG, wherein the IPG is preferably implanted in the vicinity of the proximal target tissue, i.e. in the vicinity of the occipital nerve.

According to a further embodiment, the at least one stimulation electrode may be a ring electrode which may be segmented. If more stimulation electrodes are present on the lead, one, several or all of those stimulation electrodes may be a segmented electrode. Segmented electrodes are beneficial since they allow for directional stimulation. According to yet a further embodiment, the stimulation lead may comprise an active tip electrode located directly on its distal end. In particular, the tip electrode may comprise a surface area that is larger than that of the at least one ring electrode. Furthermore, the tip electrode may be a full-body electrode or segmented. The tip electrode having a larger surface area than the ring electrodes is beneficial, since it facilitates tissue stimulation that is further away from the distal end, e.g. that is deeper inside the sphenopalatine fossa. The tip electrode may also be configured for generation of a stimulation current that can be directed towards the front of distal end of the lead.

According to a further embodiment, the middle portion of the lead, at a variable distance from the proximal end of the lead, may contain one or more additional ring electrodes for stimulation. These electrodes may be full-body electrodes or segmented electrodes. These middle electrodes may provide alternative stimulation options by acting as distance poles to a distal electrode.

According to a further embodiment, the lead holder may be configured for releasably clamping the stimulation lead. The lead holder may comprise a handle portion and a shaft portion. The shaft portion may be hollow and may serve as a stiffener or as reinforcement of the stimulation lead, since the lead itself is rather flexible in order to adjust to its surrounding tissue once implanted.

The lead holder therefore functions as a means with which the lead can be pushed or shoved into the puncture created by the second trocar. The lead holder may still be radially flexible along the longitudinal axis of its shaft portion, as long as its stiffness along its longitudinal axis is greater than that of the stimulation lead.

In order to increase the stiffness of the lead holder, in particular of the shaft portion of the lead holder, the system further may comprise a constriction suture for tightening and thereby stiffening the shaft portion along its longitudinal axis. In particular, the constriction suture may be tied or wound around the hollow shaft portion of the lead holder before the latter is inserted into the tissue. Before inserting the lead into the distal target tissue via the lead holder, the pull suture may be connected to the free end of the lead, i.e., to the proximal end of the lead, which is the end that is not clamped by the lead holder (the clamped end corresponds to the distal end of the lead). The distal end is then pushed into the guide tube and into the puncture via the lead holder until the distal end reaches the distal target tissue. The proximal end of the lead is pulled through the tunneling incision behind the ear, thereby creating forces that can barely be taken by the engaged lead tines. For this reason, the constriction suture is only severed once the proximal end of the lead has reached the proximal target position (e.g., the vicinity of the occipital nerve).

According to a further embodiment, the shaft portion of the lead holder may comprise at least one groove configured for receiving at least a portion of the constriction suture. This way, the constriction suture does not slip or loosen upon insertion of the lead holder through the introducer. Furthermore, the groove decreases the overall diameter of the constricted lead holder since the constriction suture may be at least partly embedded in the groove. This way, the design can be kept small. In addition to the above, after cutting the constriction suture, it can move freely and can be pulled from the lead holder and out of the introducer along the groove.

According to a further embodiment, the shaft portion of the lead holder may comprise a third slot-like opening that proceeds along a longitudinal axis of the shaft portion. The third slot-like opening allows for an easy release of the stimulation lead once the proximal and/or distal target tissues are reached.

According to a further embodiment, the introducer may comprise an introducer navigation array. Likewise, the tunneling tool may comprise a tunneling tool navigation array, the first trocar may comprise a first trocar navigation array, the guide tube may comprise a guide tube navigation array, and the second trocar may comprise a second trocar navigation array. Each navigation array may be detected and monitored by a navigation guidance system. Furthermore, each navigation array can be created by optical markers or electromagnetic sensors that are suitable for detection by an optical navigation system or an electromagnetic navigation system. The navigation guidance system, which may also be part of the system described herein, may preferably comprise previously generated medical images of the patient such as CT and/or MRI images. The navigation guidance system may further comprise a navigation software which assists in defining both the distal and the proximal target locations. The navigation software may also be capable of calculating the required lead length and can simulate the expectable stimulation efficacy based on the individual patient's anatomy.

According to another aspect of the invention, a method of implanting a stimulation lead in a subject's head and facial tissue is presented, wherein the method uses the system described herein and wherein the method comprises:
a) inserting the first trocar in the introducer;
b) inserting the introducer with the first trocar into the subject's facial tissue through a puncture in the subject's cheek;
c) inserting the tunneling tool into the subject's facial tissue through an incision made behind the subject's ear;
d) pushing the shaft of the tunneling tool subcutaneously in the direction of the first trocar inserted in the subject's cheek until the releasable shaft tip is retained in the receiving structure of the first trocar;
e) removing the first trocar with the retained shaft tip from the introducer until a first end of the pull suture protrudes out of the introducer;
f) removing the tunneling tool from the subject's facial tissue until a second end of the pull suture protrudes out of the incision behind the subject's ear:
g) inserting the second trocar in the guide tube and insert the guide tube with second trocar in the introducer;
h) pushing the guide tube and the second trocar until the tip of the second trocar reaches its distal target position;
i) removing the second trocar from the still inserted guide tube;
j) inserting the lead holder holding the stimulation lead and constricted by the constriction suture in the guide tube and push down on the lead holder until the distal end of stimulation lead reaches the distal target position;
k) removing the guide tube from the introducer, thereby leaving the lead holder inserted in the introducer;
l) connecting the pull suture to the connecting structure of the stimulation lead;
m) pulling on the second end of the pull suture until the proximal end of the stimulation lead reaches the proximal target position;
n) cutting constriction suture;
o) removing the lead holder from the introducer;
p) removing the introducer;
q) closing puncture and incision.

Step a) comprises preassembly of the first trocar and the introducer. The trocar is needed for puncturing the subject's cheek and for insertion of the introducer, which are both part of step b).

According to a preferred embodiment, the method may further comprise the following step: b') adjusting the disk of the introducer to a desired position and attaching the adjusted disk to the skin surface of the subject using the adhesive portion of the disk. Attaching the introducer to the subject's skin using the adhesive portion of the disk is advantageous, since it locks the position, i.e. the depth and the rotational angle of the introducer with respect to the subject's face, thereby allowing the physician two perform other task with both hands free. Locking the introducer in a position for all following steps of the implantation method is crucial, in particular since the implantation procedure may take a couple of minutes or even hours and a change of position of the introducer (such as a shift, slip or rotation) may lead to inaccurate positioning of the stimulation lead.

Steps c) to m) can partly inserting the tunneling tool into the subject's facial tissue through an incision made behind the subject's ear;
d) pushing the shaft of the tunneling tool subcutaneously in the direction of the first trocar inserted in the subject's cheek until the releasable shaft tip is retained in the receiving structure of the first trocar;
e) removing the first trocar with the retained shaft tip from the introducer until a first end of the pull suture protrudes out of the introducer;
f) removing the tunneling tool from the subject's facial tissue until a second end of the pull suture protrudes out of the incision behind the subject's ear:
g) inserting the second trocar in the guide tube and insert the guide tube second trocar in the introducer;
h) pushing the guide tube and the second trocar until the tip of the second trocar reaches its distal target position;
i) removing the second trocar from the still inserted guide tube;
j) inserting the lead holder holding the stimulation lead and constricted by the constriction suture in the guide tube and push down on the lead holder until the distal end of stimulation lead reaches the distal target position;
k) removing the guide tube from the introducer, thereby leaving the lead holder inserted in the introducer;
l) connecting the pull suture to the connecting structure of the stimulation lead;

According to a further embodiment, the method may further comprise the following step:
n') after step m) or after step n), connecting the stimulation lead to an implanted pulse generator (IPG) implanted in the subject's head and facial tissue. The IPG may already comprise one or several stimulation leads already attached or connected to the IPG. This way, stimulation of two or more tissues, e.g. of the SPG and the occipital nerve, may be performed using only one IPG. This "dual" stimulation may further be performed simultaneous or in an alternating manner so as to provide stimulation that may abort and/or prevent symptomatic events.

According to a further embodiment, the method may further comprise the following step:
h') after step h), locking the guide tube in the introducer using the lock mechanism, thereby the fixing guide tube's position with respect to the introducer.

According to a further embodiment, the method may further comprise the following step:
j') after step j), locking the lead holder in the introducer using the lock mechanism, thereby fixing the lead holder's position with respect to the introducer.

According to a further embodiment, the method may further comprise the following step:
a') before step c), applying anesthetics to the head and facial tissue in which the stimulation lead is to be implanted.

According to a further embodiment, one or more of the steps a'), a), b), c), d) and/or h) may be performed using the navigation guidance system.

Unless stated otherwise, the method steps described above do not necessarily have to be performed in alphabetical order or in the order at which they are presented.

Any one of the embodiments, examples or features disclosed herein may be used in combination or separately and in conjunction with any one of the aspects of the disclosed subject matter, mutatis
mutandis.

### Detailed description of the invention

The accompanying drawings, which are incorporated in and constitute a part of this disclosure, illustrate several examples of the described subject matter. The drawings show the following:
- Fig. 1a-e: depict schematic illustrations of different parts of a system for implanting a stimulation lead into a subject's body according to a preferred embodiment;
- Fig. 2: depicts a schematic illustration of a stimulation lead according to a preferred embodiment;
- Fig. 3a-e: depict schematic illustrations of stimulation leads according to different embodiments;
- Fig. 4a-e: depict a method of implanting a stimulation lead into a subject's body using a system as disclosed herein;
- Fig. 5: depicts an implanted stimulation lead that is connected to an IPG.

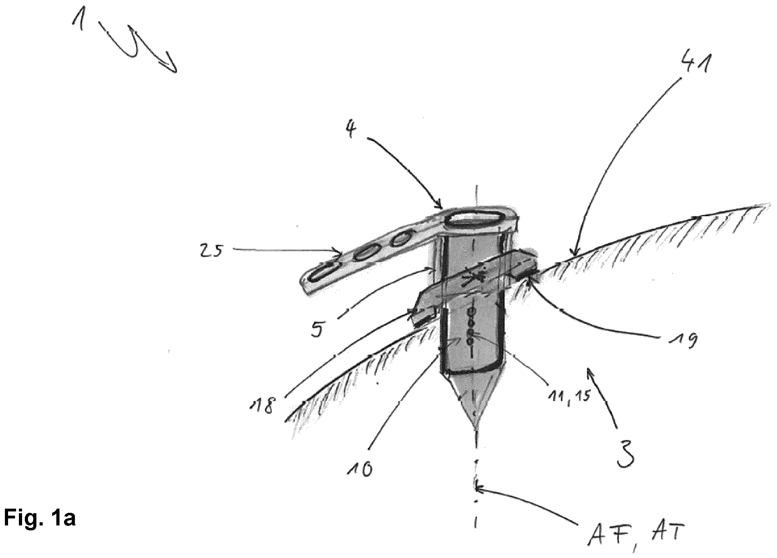

Fig. 1a to Fig. 1e show a schematic illustration of a system 1 for implanting a stimulation lead 2 into a subject's body according to a preferred embodiment. Fig. 1a shows a part of the system 1. In particular, Fig. 1a shows an introducer 4 configured for temporary insertion into a head and facial tissue 3 beneath a subject's cheek 41. The Introducer 4 comprises a substantially hollow tube portion 5 and a navigation array 25 attached thereto. As is shown in Fig. 1a, the system 1 also comprises a first trocar 10 configured for insertion into the introducer 4. In fact, the first trocar 10 is already inserted in the introducer 4 and partly inserted in the subject's tissue 3. The first trocar 10 comprises a receiving structure 11 for receiving and retaining the shaft tip 9 of the tunneling tool 6 (not shown in Fig. 1a). As is depicted in Fig. 1a, the receiving structure 11 may comprise several snap-in openings 15 arranged along the longitudinal axis AF of the first trocar 10. This pattern of longitudinally arranged snap-in openings 15 has the advantage that the first trocar may receive the shaft tip 9 at different depths of tissue 3 after insertion.

The tube portion 5 of the introducer 4 may also comprise a first slot-like opening 16 that proceeds along its longitudinal axis AT (not shown in Fig. 1a). This allows the shaft tip 9 of the tunnelling tool 6 to meet and connect with the receiving structure 11 of the first trocar 10. Together with the introducer 4, the first trocar 10 is configured for making a puncture in the subject's cheek 41 to reach a certain tissue 3 depth. Both the introducer 4 and the first trocar 10 are inserted into the subject's cheek 41 along their respective axes AF and AT, which coincide in the instance depicted in Fig. 1a.

The introducer 4 shown in Fig. 1a comprises a disk 18 having an adhesive portion 19, wherein the adhesive portion 19 of the disk 18 is attached to the subject's cheek 41. The disk 18 is tilted with respect to the longitudinal axis AT of the tube portion 5 of the introducer 4 and thereby locks or fixes the introducer 4 its current position with respect to the subject's cheek 41.

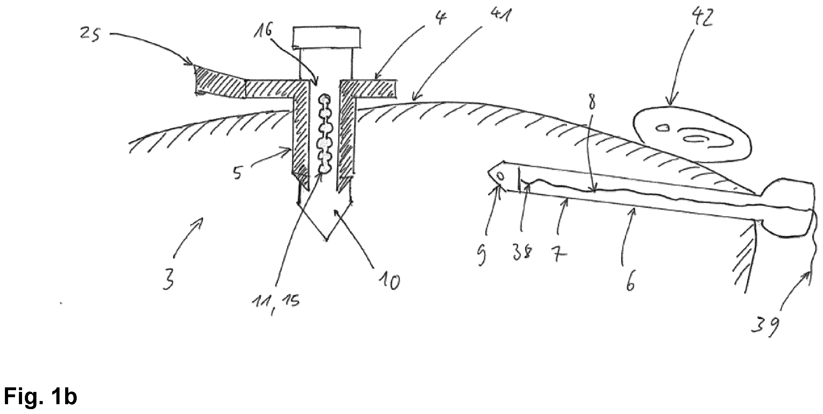

Fig. 1b depicts a schematic illustration of another part of the system shown in Fig. 1a. In particular, Fig. 1b shows, in addition to Fig. 1a, the tunneling tool 6, which is configured for temporary insertion into the subject's tissue 3 from an incision behind the ear 42 and which is depicted as inserted in said tissue in Fig. 1b. The tunneling tool 6 comprises a shaft 7, a shaft tip 9 releasably mounted to the shaft 7, and a pull suture 8 connected to the releasable shaft tip 9. The pull suture 8 has a first end 38 (the end connected to the shaft tip) and a second end 39, which extends outwards from the incision behind the subject's ear 42. The sizes of the tools depicted as well as tissue depths are not necessarily to scale. In particular, the shaft 7 of the tunneling tool 6 is designed in such a way that the shaft tip 9 can easily reach the receiving structure 11 of the first trocar 10 when the latter is inserted in the tissue 3 beneath the subject's cheek 41.

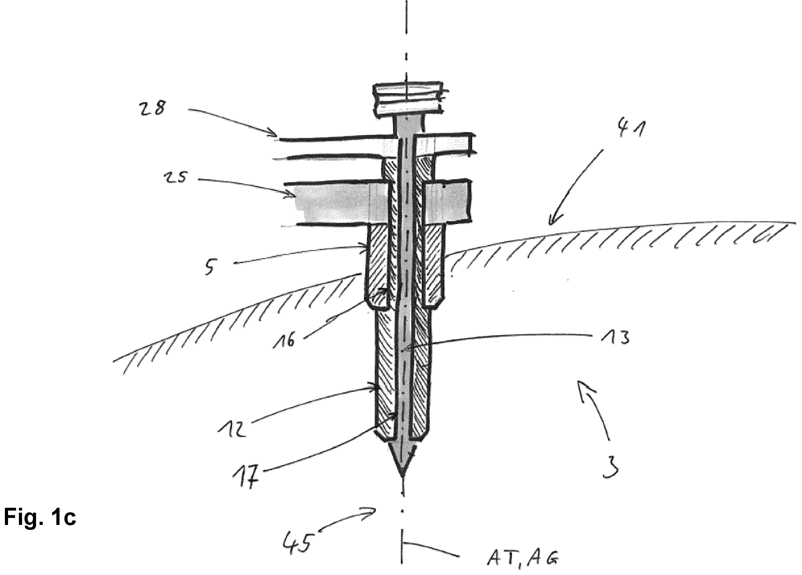

Fig. 1c depicts a schematic illustration of another part of the system 1 shown in Fig. 1c. In particular, Fig. 1c depicts a guide tube 12, which comprises a navigation array 28 and is configured for insertion into the introducer 4 and a second trocar 13, which is configured for insertion into the guide tube 12. As shown in Fig. 1c, the first trocar 10 has been removed from the introducer 4 and instead both the guide tube 12 and the second trocar 13 have been inserted through the introducer 4 in the subject's tissue 3. The disk 18 is not shown in Fig. 1c. The second trocar 13 is designed in such a way that it reaches a bigger tissue 3 depth than the first trocar 10. In particular, the second trocar 13 can reach a distal target position 45, which can for example be the tissue surrounding the SPG or the sphenopalatine fossa.

As is further depicted in Fig. 1c, the tube portion 5 of the introducer 4 comprises a first slot-like opening 16 that proceeds along its longitudinal axis AT. Furthermore, the guide tube 12 comprises second slot-like opening 17 that proceeds along its longitudinal axis AG. The tube portion 5 and the guide tube 12 are furthermore shaped in such way that the guide tube 12 only fits inside the tube portion 5 of the introducer 4 in one orientation. This can be important, since it guarantees that the first slot-like opening 16 of the tube portion 5 and the second slot-like opening 17 of the guide tube 12 can coincide. The slot-like opening 17 of the guide tube 12 may be particularly advantageous once the lead holder 14 (not shown in Fig. 1c) is inserted into the guide tube 12. In particular, the second slot-like opening 17 facilitates removal of the guide tube 12 from the introducer 4 while at the same time leaving the lead holder 14 inserted in the tube portion 5 of the introducer 4.

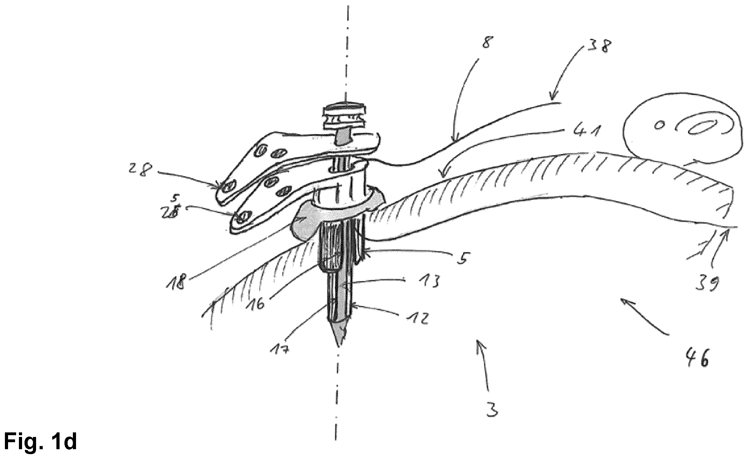

Fig. 1d depicts a schematic illustration of a similar part of the system 1 as shown in Fig. 1c above. In Fig. 1d, however, the disk 18 attached to the subject's cheek 41 skin is depicted. Furthermore Fig. 1d shows the pull suture 8 after being pulled through the introducer 4 by using the first trocar 10. As is shown in Fig. 1d, the first end of the pull suture 8 extends outwards through the puncture in the subject's cheek 41 and through the introducer 4, and the second end 39 of the pull suture 8 extends outwards of the incision behind the subject's ear 42.

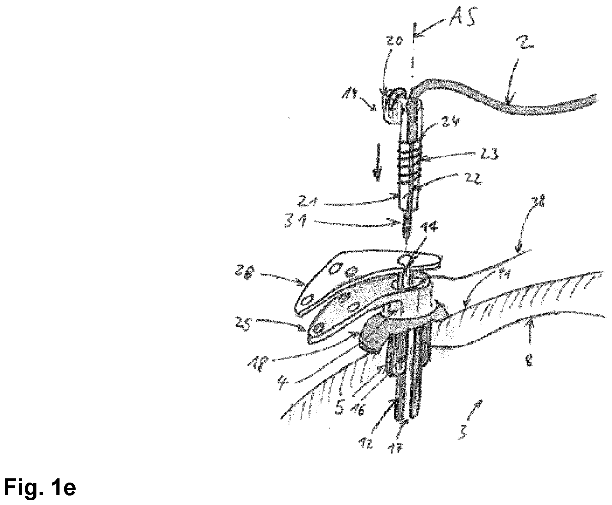

Fig. 1e depicts a schematic illustration of yet another part of said system 1. The system 1 part shown in Fig. 1e differs from the system 1 part shown in Fig. 1d in that the system as shown in Fig. 1e further comprises a lead holder 14 and a stimulation lead 2. The lead holder 14 is configured for retaining the lead 2 into the subject's head and facial tissue 3 through the guide tube 12 inserted in the introducer 4 inserted in the subject's cheek 41.

The lead holder 14 shown in Fig. 1e comprises a handle portion 20 and a hollow shaft portion 21. The shaft portion 21 holds the stimulation lead 2 and serves as a stiffener or as reinforcement for the lead 2. The lead 2 can be pushed through the puncture created by the second trocar 13 using the lead holder 14 until the distal end of the lead 2 reaches its distal target position 45 (not shown in Fig. 1e). Therefore, the lead holder 2 should still be radially flexible along the longitudinal axis of the shaft portion AS, as long as its stiffness along the longitudinal axis AS is greater than that along the longitudinal axis AL of the stimulation lead 2.

As shown in Fig. 1e, the lead holder 14 holding the stimulation lead 2 is additionally supported by a constriction suture 23, which is tied or rather wound around the shaft portion 21 of the lead holder 14, wherein both ends of the constriction suture 23 preferably meet at the handle portion 20 of the lead holder 14. Although not clearly visible in Fig. 1e, the shaft portion 21 of the lead holder 14 also comprises a groove 24configured for receiving at least a portion of the constriction suture 23. This way, the constriction suture 23 is prevented from slipping or loosening upon insertion of the lead holder 14 through the introducer 4. Furthermore, the groove 24 decreases the overall diameter of the constricted lead holder 14, since the constriction suture 23 may be at least partly embedded in the groove 24.

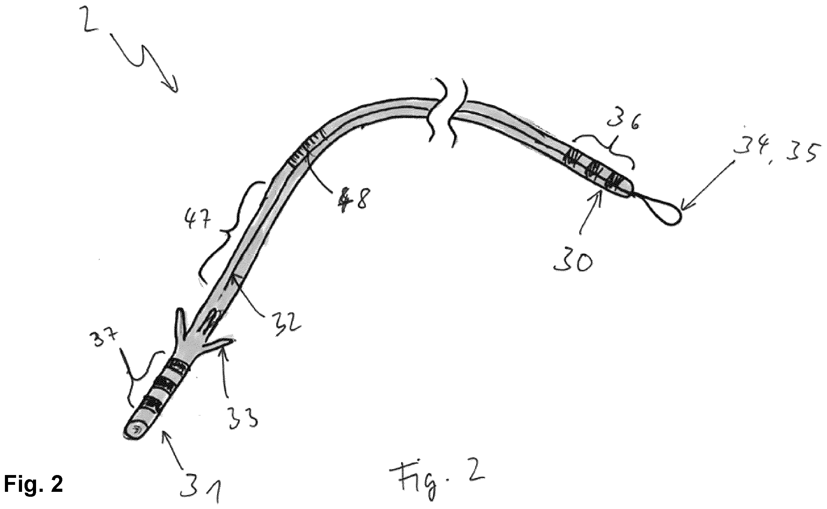

Fig. 2 depicts a schematic illustration of the stimulation lead 2 according to a preferred embodiment. The lead 2 shown in Fig. 2 has a proximal end 30 configured for implantation at the proximal target position 46 and distal end 31 configured for implantation at the distal target position 45. At its distal end 31, the stimulation lead 2 comprises three stimulation electrodes 37, which are designed as ring electrodes. The distal end of the stimulation lead 2 further comprises two pairs of tines 33 which are equidistantly located around the distal end 31, making for a total of four tines 33. The tines 33 can engage with the tissue 3 in the distal target position 45 and thereby guarantee that the stimulation lead 2 stays in its position after implantation.

At its proximal end 30, the lead 2 comprises three further ring electrodes. However, the ring electrodes located at the proximal end 30 of the lead are connector electrodes 36 configured for connection with an already implanted IPG, wherein the IPG is preferably implanted in the vicinity of the proximal target tissue 46, i.e. in the vicinity of the occipital nerve. The proximal end 30 of the simulation lead 2 further comprises a connection structure 34, which in embodiment shown in Fig. 2 is a looped strand 35. The connection structure 34 serves as a structure to which the first end 38 of the pull suture 8 can be connected, e.g. tied or knotted, once the pull suture 8 has been tunneled through the subject's cheek 41. The connection structure 34, i.e. the looped strand 35, is further connected to an internal strain relief incorporated along the longitudinal axis AL of the lead 2. The strain relief protects the integrity of the stimulation lead 2, i.e. it protects the lead's 2 internal wires from damaging while the proximal end 30 of the lead 2 is pulled in the direction of the incision behind the subject's ear 42.

Between its two ends 30, 31, the stimulation lead 2 has a middle portion 49 which comprises a scale 48 having markings in order to guarantee correct insertion depth of the distal 31 end of the lead 2 during implantation procedure.

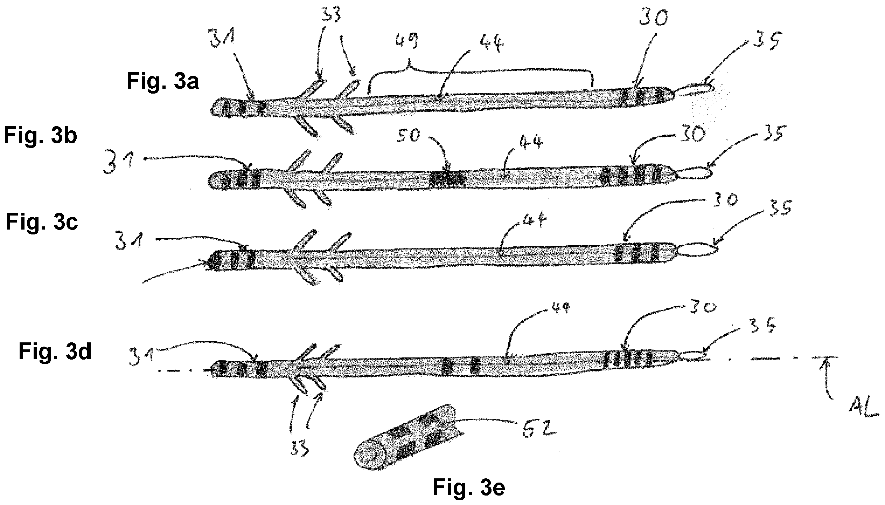

Fig. 3a-e depict schematic illustrations of further stimulation leads 2 according to different embodiments. All leads 2 shown comprise a proximal end 30 and a distal end 31 connected by a middle portion 49. Furthermore, the distal ends 31 of the leads 2 comprise two pairs of tines 33 located along the longitudinal axis AL of the stimulation lead 2. All leads 2 shown in Fig. 3a-e also comprise a looped strand 35 connected to a strain relief 44. The distal ends 31 of all shown leads 2 comprise three stimulation electrodes 37. The proximal ends 30 of all shown leads 2 either comprise three (Fig. 3b and Fig. 3d) or four (Fig. 3a and Fig. 3c) connector electrodes 38.

In addition to the above, the stimulation lead depicted in Fig. 3b comprises a middle portion electrode 50 located in the middle portion 49 of the lead 2. The middle portion electrode 50 has a larger surface area than the stimulation electrodes 37 located at the distal end of the lead 2 and is configured for generation of a virtual monopolar stimulation paradigm.

The stimulation lead 2 depicted in Fig. 3c comprises an active tip electrode 51 located directly on the distal end 31. This tip electrode 51 is configured for generation of a stimulation current that can be directed towards the front of distal end of the lead 2.

Fig. 3d comprises two additional ring electrodes located in the middle portion 49 of the stimulation lead. This allows parallel stimulation of further facial nerves, e.g. of the maxillary nerve.

Fig. 3e depicts an alternative design of the ring electrodes option to ring electrodes, i.e. segmented electrodes 52. These segmented electrodes 52, also named directional electrodes, are configured for generation of directed stimulation pulses.

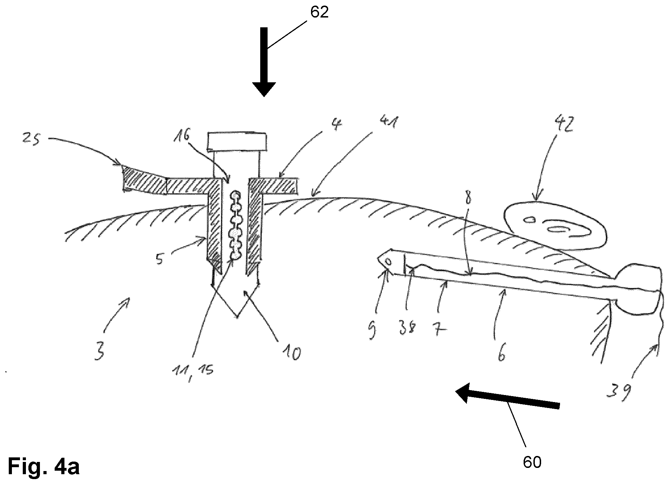

Fig. 4a-e depict a method of implanting a stimulation lead into a subject's body using a system 1 as disclosed herein according to a preferred embodiment.

As shown in Fig. 4a, which corresponds to Fig. 1b, the first trocar 10 has been inserted in the introducer 4 (step a) and both the trocar 10 and introducer 4 have been inserted in a downward direction 62 into the subjects' tissue 3 through a puncture in the subject's cheek 41 (step b). In addition, the tunneling tool 6 has been inserted into the subject's facial tissue 3 through an incision made behind the subject's ear 42 (step c). The shaft 7 of the tunneling tool 6 is pushed subcutaneously in an inward direction 60, i.e. in the direction of the first trocar 10, until the releasable shaft tip 90 is retained in the receiving structure 11 of the first trocar 10 (step d). The sizes of the tools depicted as well as tissue depths are not necessarily to scale. In particular, the shaft 7 of the tunneling tool 6 is designed in such a way that the shaft tip 9 can easily reach the receiving structure 11 of the first trocar 10 when the latter is inserted in the tissue 3 beneath the subject's cheek 41.

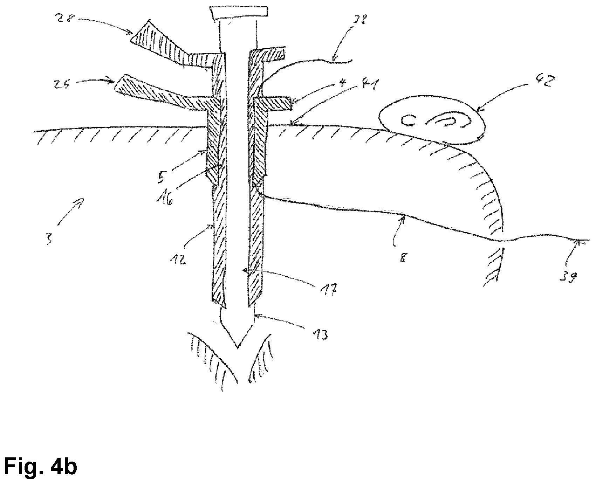

Fig. 4b depicts the procedure during or after steps e)-h). The first trocar 10 is removed from the introducer 4 with the shaft tip 9 retained in its receiving structure 11 until the first end 28 of the pull suture 8 protrudes out of the introducer 4 (step e). Also, the tunneling tool 6 is removed from the subject's facial tissue 3 until the second end 39 of the pull suture 6 protrudes out of the incision behind the subject's ear 42 (step f). Once the first trocar 10 is removed from the introducer 4, the second trocar 13 is slidably inserted in the guide tube 12. Then, the guide tube 12 inserted in the introducer 4 together with the second trocar 13. The guide tube 12 and the second trocar 13 are then pushed into the subject's cheek until the tip of the second trocar 13 reaches its distal target position 45.

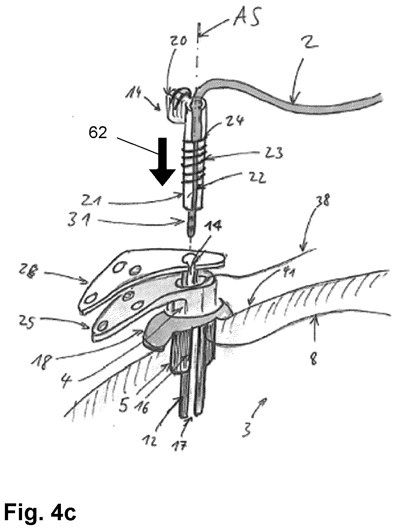

Fig. 4c depicts the procedure during or after steps i)-j). In particular, it shows the steps after removal of the second trocar 13 from the still inserted guide tube 12 (step i). Furthermore, it shows the insertion of the lead holder 14 holding the stimulation lead 2, constricted by the constriction suture 23, into the guide tube 12 (step j). As depicted, the lead holder 14 is inserted into the guide 12 in a downward direction 62.

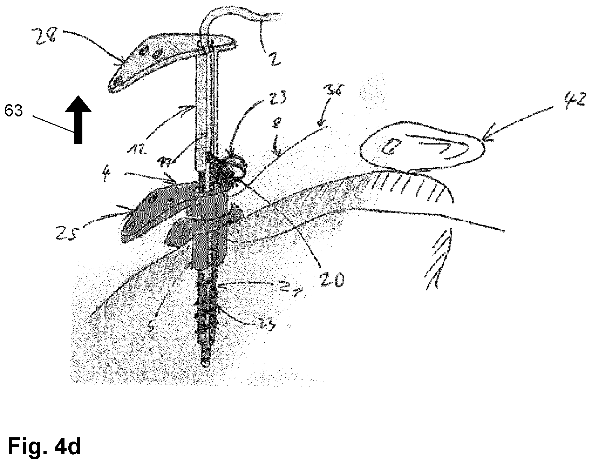

Fig. 4d depicts the procedure during step k). In this step, the guide tube 12 is removed from the introducer 4, while leaving the lead holder 14 still inserted. The slot 17 in the guide tube 12 allows for removal of the guide tube 12 by slide it over the lead holder 14 in an upward direction 63 without having to remove the lead holder 14 from the introducer 4. Thus, after step k), only the lead holder 14 holding the stimulation lead 2 is still inserted in the introducer 4 and therefore in the subject's cheek 41.

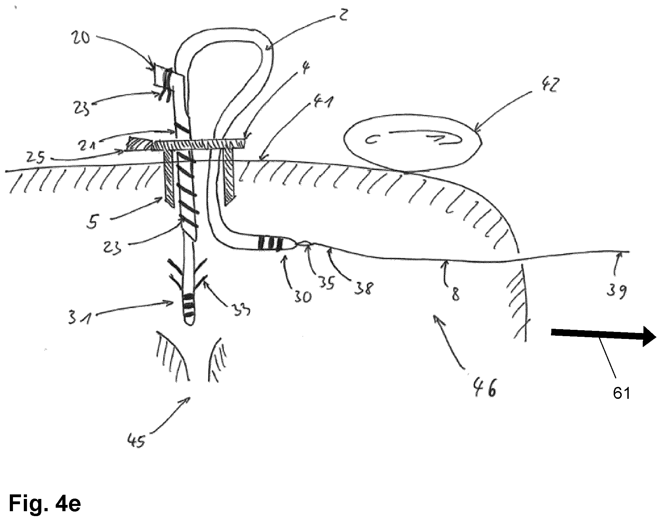

Lastly, Fig. 4e shows steps l)-m) of the procedure. In particular, the pull suture 8 is tied to the looped strand 35 of the stimulation lead 2 (step I). Afterwards, second end 39 of the pull suture 8 is pulled upon in an outward direction 61 until the proximal end 30 of the stimulation lead 2 reaches the proximal target position 46.

Afterwards, the constriction suture 23 is cut to release the lead 2 from the lead holder 14 once tunneling is finished. The constriction suture 23 is accessible from the most proximal section of the lead holder 14, e.g. the handle portion 20, to allow cutting the suture 23 from outside the subject's body. The lead holder 14 is subsequently removed from the introducer 4 and the introducer 4 is removed from the subject's cheek's 41 tissue 3.

Although not shown in Fig. 4a-e, some or all steps of the depicted method may be performed using the navigation guidance system. Furthermore, local and/or general anesthetics may have been applied to subject prior to the implantation steps.

Unless stated otherwise, the method steps described above do not necessarily have to be performed in alphabetical order or in the order at which they are presented.

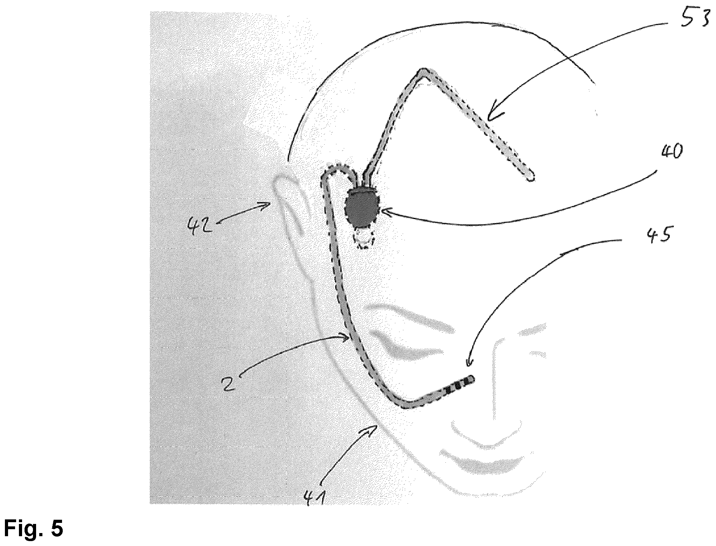

Fig. 5 depicts a stimulation lead 2 that is implanted in a subject's head and facial tissue and that is electrically connected to an IPG 40, wherein the IPG 40 comprises a second lead 53 that is also electrically connected to the IPG 40. The stimulation lead 2, the IPG 40 and the second lead 53 are depicted in dashed lines to indicate that the are implanted in the subject's head and facial tissue. With a neurostimulation system as shown in Fig. 5, stimulation of two or more tissues can be performed using only one IPG. In case of Fig. 5, the different tissues to be stimulated are the SPG and the occipital nerve. This so-called dual stimulation may be performed in a simultaneous or in an alternating manner so as to provide stimulation that may abort and/or prevent symptomatic events.

Any one of the embodiments, examples or features disclosed herein may be used in combination or separately and in conjunction with any one of the aspects of the disclosed subject matter, mutatis mutandis.

### List of reference numerals

| | | | |
|---|---|---|---|
| 1 | System | 34 | Connecting structure |
| 2 | Neurostimulation lead | 35 | Looped strand |
| 3 | Head and facial tissue | 36 | Connector electrode |
| 4 | Introducer | 37 | Stimulation electrode |
| 5 | Tube portion | 38 | First end |
| 6 | Tunneling tool | 39 | Second end |
| 7 | Shaft | 40 | Implanted pulse generator |
| 8 | Pull suture | 41 | Cheek |
| 9 | Shaft tip | 42 | Ear |
| 10 | First trocar | 43 | Head portion |
| 11 | Receiving structure | 44 | Strain relief |
| 12 | Guide tube | 45 | Distal target position |
| 13 | Second trocar | 46 | Proximal target position |
| 14 | Lead holder | 47 | Clamp segment |
| 15 | Snap-in openings | 48 | Scale markings |
| 16 | First slot-like opening | 49 | Middle portion |
| 17 | Second slot-like opening | 50 | Middle portion electrode |
| 18 | Disk | 51 | Tip electrode |
| 19 | Adhesive portion | 52 | Segmented electrodes |
| 20 | Handle portion | 53 | Second lead |
| 21 | Shaft portion | | |
| 22 | Third slot-like opening | 60 | Inward direction |
| 23 | Constriction suture | 61 | Outward direction |
| 24 | Groove | 62 | Downward direction |
| 25 | Introducer navigation array | 63 | Upward direction |
| 26 | Tunneling tool navigation array | | |
| 27 | First trocar navigation array | AF | Longitudinal axis of the first trocar |
| 28 | Guide tube navigation array | AT | Longitudinal axis of the tube portion |
| 29 | Second trocar navigation array | AG | Longitudinal axis of the guide tube |
| 30 | Proximal end | AS | Longitudinal axis of the shaft portion |
| 31 | Distal end | AL | Longitudinal axis of the lead |
| 32 | Strain relief | | |
| 33 | Tines | | |

## Claims

1. System (1) for implanting a neurostimulation lead (2) in a subject's head and facial tissue (3), the system (1) comprising:
a stimulation lead (2) configured for implantation in said tissue (3);
an introducer (4) configured for temporary insertion into said tissue (3) and comprising a Tube portion (5);
a tunneling tool (6) configured for temporary insertion into said tissue (3) and comprising a shaft (7), a shaft tip (9) releasably mounted to the shaft (7), and a pull suture (8) connected to the releasable shaft tip (9);
a first trocar (10) configured for insertion into the introducer (4), the first trocar (10) comprising a receiving structure (11) for receiving and retaining the shaft tip (9) of the tunneling tool (6);
a guide tube (12) configured for insertion into the introducer (4);
a second trocar (13) configured for insertion into the guide tube (12);
and a lead holder (14) configured for inserting the stimulation lead (2) through the guide tube (12) into said tissue.

2. System (1) according to claim 1, **characterized in that** the shaft tip (9) of the tunneling tool (6) is configured for snapping into the receiving structure (11) of the first trocar (10) wherein the receiving structure (11) preferably comprises several snap-in openings (15) arranged along a longitudinal axis (AF) of the first trocar (10).

3. System (1) according to any of the preceding claims, **characterized in that** the tube portion (5) of the introducer comprises a first slot-like opening (16) that proceeds along a longitudinal axis (AT) of the tube portion (5).

4. System (1) according to any of the preceding claims, **characterized in that** the guide tube (12) comprises a second slot-like opening (17) that proceeds along a longitudinal axis (AG) of the guide tube (12).

5. System (1) according to any of the preceding claims, **characterized in that** the introducer (4) comprises a lock mechanism for locking the guide tube (12) and/or the lead holder (14) in the introducer (4) when the guide tube (12) and/or the lead holder (14) reached its desired position in the subject's head and facial tissue (3).

6. System (1) according to any of the preceding claims, **characterized in that** the introducer (4) comprises a disk (18) having an adhesive portion (19) wherein the adhesive portion (19) of the disk (18) is configured for attachment to the subject's skin wherein the disk (18) is preferably tiltable with respect to a longitudinal axis (AT) of the tube portion (5) and/or rotatable around the longitudinal axis (AT) of the tube portion (5), and wherein the disk (18) is preferably tiltable and/or rotatable in discrete increments.

7. System (1) according to any of the preceding claims, **characterized in that** the lead holder (14) comprises a handle portion (20) and a shaft portion (21) and wherein the shaft portion (21) of the lead holder (14) preferably comprises a third slot-like opening (22) that proceeds along a longitudinal axis (AS) of the shaft portion (21).

8. System (1) according to claim 7, **characterized in that** the system (1) further comprises a constriction suture (23) for tightening and thereby stiffening the shaft portion (21) along its longitudinal axis (AS).

9. System (1) according to claim 8, **characterized in that** the shaft portion (21) of the lead holder (14) comprises at least one groove (24) configured for receiving at least a portion of the constriction suture (23).

10. System (1) according to any of the preceding claims, **characterized in that** the introducer (4) comprises an introducer navigation array (25).

11. System (1) according to any of the preceding claims, **characterized in that** the tunneling tool (6) comprises a tunneling tool navigation array (26).

12. System (1) according to any of the preceding claims, **characterized in that** the guide tube (12) comprises a guide tube navigation array (28).

13. System (1) according to any of the preceding claims, **characterized in that** the stimulation lead (2) comprises an internal strain relief (32) to protect the integrity of the stimulation lead (2).

14. System (1) according to any of the preceding claims, **characterized in that** the stimulation lead (2) comprises at least one pair of tines (33) located towards its distal end (31).

15. System (1) according to any of the preceding claims, **characterized in that** the stimulation lead (2) comprises at its proximal end (30) a connecting structure (34) to which the pull suture (8) is connectable, wherein the connecting structure (34) is preferably a looped strand (35).

16. System (1) according to claim 15, **characterized in that** the stimulation lead (2) comprises at least one connector electrode (36) at its proximal end (30) and at least one stimulation electrode (37) at its distal end (31), wherein the stimulation lead (2) preferably comprises at least three connector electrodes (36) at its proximal end (30) and at least three stimulation electrodes (37) at its distal end (31).

17. System (1) according to claim 16, **characterized in that** the at least one connector electrode (36) and/or the at least one stimulation electrode (37) is a ring electrode, wherein the ring electrode is preferably a segmented electrode (52).

18. Method of implanting a stimulation lead (2) in a subject's head and facial tissue (3) using the system (1) according to any one of the preceding claims, the method comprising the following steps:
a) inserting the first trocar (10) in the introducer (4);
b) inserting the introducer (4) with the first trocar (10) into the subject's facial tissue through a puncture in the subject's cheek (41);
c) inserting the tunneling tool (6) into the subject's facial tissue through an incision made behind the subject's ear (42);
d) pushing the shaft (7) of the tunneling tool (6) subcutaneously in the direction of the first trocar (10) inserted in the subject's cheek (41) until the releasable shaft tip (9) is retained in the receiving structure (11) of the first trocar (10);
e) removing the first trocar (10) with the retained shaft tip (9) from the introducer (4) until a first end (38) of the pull suture (8) protrudes out of the introducer (4);
f) removing the tunneling tool (6) from the subject's facial tissue until a second end (39) of the pull suture (8) protrudes out of the incision behind the subject's ear (42):
g) inserting the second trocar (13) in the guide tube (12) and insert the guide tube (12) second trocar (13) in the introducer (4);
h) pushing the guide tube (12) and the second trocar (13) until the tip of the second trocar (13) reaches the distal target position;
i) removing the second trocar (13) from the guide tube (12);
j) inserting the lead holder (14) holding the stimulation lead (2) and constricted by the constriction suture (23) in the guide tube (12) and push down on the lead holder (14) until the distal end (31) of stimulation lead (2) reaches the distal target position;
k) removing the guide tube (12) from the introducer (4), thereby leaving the lead holder (14) inserted in the introducer (4);
l) connecting the pull suture (8) to the connecting structure (34) of the stimulation lead (2);
m) pulling on the second end (39) of the pull suture (8) until the proximal end (30) of the stimulation lead reaches the proximal target position;
n) cutting constriction suture (23):
o) removing the lead holder (14) from the introducer (4);
p) removing the introducer (4);
q) closing puncture and incision.

19. Method according to claim 18, **characterized in that** the method further comprises the following step:
m') after step m), connecting the stimulation lead (2) to an implanted pulse generator (40) implanted in the subject's head and facial tissue (3).

20. Method according any of claims 18 or 19, **characterized in that** the method further comprises the following step:
b') after step b), adjusting the disk (18) of the introducer (4) to a desired position and attaching the adjusted disk (18) to the skin surface of the subject using the adhesive portion (19) of the disk (18).

21. Method according any of claims 18 to 20, **characterized in that** the method further comprises the following step:
h') after step h), locking the guide tube (12) in the introducer (4) using the first lock mechanism, thereby the fixing guide tube's (12) position with respect to the introducer (4).

22. Method according any of claims 18 to 21, **characterized in that** the method further comprises the following step:
j') after step j), locking the lead holder (14) in the introducer (4) using the second lock mechanism, thereby fixing the lead holder's (14) position with respect to the introducer (4).

23. Method according any of claims 18 to 22, **characterized in that** one or more of the steps a'), a), b), c), d) and/or h) are performed using the navigation guidance system.
